(19) **Europäisches Patentamt**

**European Patent Office**

**Office européen des brevets**

(11) **EP 0 941 707 B1**

(12) **EUROPEAN PATENT SPECIFICATION**

(45) Date of publication and mention
of the grant of the patent:
**04.05.2005 Bulletin 2005/18**

(51) Int Cl.7: **A61C 1/00**, A61B 18/20

(21) Application number: **99830134.5**

(22) Date of filing: **12.03.1999**

(54) **Method and device for controlling temperature in laser treatment in dentistry**

Verfahren und Vorrichtung zur Steuerung der Temperatur bei einer zahnärztlichen Laserbehandlung

Méthode et dispositif pour contrôler la température lors d'un traitement dentaire au laser

(84) Designated Contracting States:
**AT BE CH DE DK ES FI FR GB GR IE IT LI LU MC NL PT SE**

(30) Priority: **13.03.1998 IT FI980059**

(43) Date of publication of application:
**15.09.1999 Bulletin 1999/37**

(73) Proprietor: **EL.EN. S.p.A.**
**50041 Calenzano (Firenze) (IT)**

(72) Inventors:
• **Clementi, Gabriele**
**50139 Firenze (IT)**
• **Modi, Stefano**
**50032 Borgo San Lorenzo (Firenze) (IT)**
• **Sieni, Alessandro**
**50018 Scandicci (Firenze) (IT)**

(74) Representative: **Mannucci, Michele et al**
**Ufficio Tecnico Ing.A. Mannucci,**
**Via della Scala 4**
**50123 Firenze (IT)**

(56) References cited:
**WO-A-96/34569**          **US-A- 5 662 643**

• **PATENT ABSTRACTS OF JAPAN vol. 1998, no. 06, 30 April 1998 (1998-04-30) -& JP 10 033548 A (MATSUSHITA ELECTRIC IND CO LTD), 10 February 1998 (1998-02-10) -& DATABASE WPI Section PQ, Week 199816 Derwent Publications Ltd., London, GB; Class P31, AN 1998-172425 XP002200496 & JP 10 033548 A (MATSUSHITA ELECTRIC IND CO LTD), 10 February 1998 (1998-02-10)**

**Description**

Technical field

The present invention relates to a device for laser treatment in dentistry and a control method for this treatment, capable of making the use of the device safer and easier when used on hard tissues such as enamel and dentine.

Background art

The interaction of lasers with materials, including biological substances, is accompanied to varying degrees by the supply of heat to, and consequently an increase in the temperature of the body struck by the laser energy. In the field of dentistry, lasers are also used to treat the hard tissues, namely enamel and dentine.

It is known that the living tooth cannot withstand temperature increases in excess of certain known values above the base level without suffering damage to the pulp tissue, with consequent inflammation of this tissue.

At present, a dentist using a laser does not have access to means for directly measuring the temperature of the tooth. This makes the use of lasers in these applications difficult and slow. This is because the dentist is obliged to apply the laser according to his judgment, in other words for sufficiently short periods and with a sufficiently long interval between one application and the next to prevent hazardous rises in the temperature of the tooth.

Japanese patent no JP 10033548 discloses a device for dental laser treatment where the treatment parameters are set by preliminary irradiating a sample and measuring the temperature of said sample.

Objects and summary of the invention

The object of the present invention is to provide a laser device for the treatment of tissues, and in particular a device for orthodontic use, which can be used to overcome the prior art problems described above.

More particularly, the object of the present invention is to provide a device which enables the operator to apply the laser without the need to intervene in an empirical and uncertain way with successive interruptions to prevent the risk of overheating of the treated tissues.

A further object of the present invention is to provide a control method for laser treatment in dentistry which enables overheating of the treated tissue to be prevented.

Yet another object of the present invention is to provide a method capable of making the use of a laser device for orthodontic applications simpler and safer, particularly in the treatment of hard tissues such as enamel and dentine.

These and other objects and advantages, which will be apparent from the following text to a person skilled in the art, are essentially achieved with a method for controlling the internal temperature of a tooth subjected to a laser beam treatment during said treatment, in which an estimated internal temperature of the tooth is determined as a function of at least one operating parameter of the laser and of the treatment time, and in which the estimated temperature is compared with a threshold temperature, the exceeding of said threshold temperature causing an alarm condition.

The alarm situation may be indicated to the operator by means of an acoustic or optical signal or by a combination of the two, or by another suitable method. There is no reason why automatic interruption of the treatment in case of alarm should not be provided.

By implementing this method in the laser treatment device, it is possible to provide the operator with a reliable and sufficiently precise estimate of the variation of temperature inside the tooth. The operator will be informed by the device when this temperature exceeds a threshold value, and will no longer be obliged to operate in an empirical and random way. This makes the device safer and much simpler to use, since all the risk situations due to the distraction of the operator will be eliminated. The estimate of the internal temperature of the tooth, which increases as a result of the supply of energy by the laser, is produced in an objective way on the basis of parameters set at the start of the treatment, and consequently the treatment is interrupted if, and only if, this is actually necessary.

It is also possible to provide for the emission of an enabling signal (or end of alarm signal) when, following an interruption of the power supply, the estimated temperature has fallen sufficiently below the alarm limit value. Alternatively, or in combination with the foregoing, it is possible to provide for the laser source to be automatically cut off for the time required to make the temperature fall below a certain value with respect to the limit value.

In a particular embodiment of the method according to the present invention, the estimated temperature is also determined as a function of the emission power of the laser. It is also possible to provide for the characteristics of the tooth, such as the type of tooth (incisor, canine, molar, premolar), its size and its age, in other words the patient's age, to be considered in the determination of the estimated temperature. The way in which these variables may affect the calculation of the estimated temperature will be described with reference to an example of embodiment.

In a particularly simple embodiment of the invention, it is possible to simply provide for the emission of an alarm signal and/or the interruption of the emission of the laser beam. However, in order to provide the operator with more information, and also to make the operation simpler and safer, in a preferred embodiment of the invention the estimated temperature is made to be shown on a graph as a function of the treatment. The operator can thus also determine (in a qualitative way at least) the time available before the limit temperature is reached. This may be useful in some circumstances.

Further advantageous embodiments and details of the execution of the method according to the invention, and of a device according to the invention, are described below and indicated in the attached claims.

Brief description of the drawings

The invention will be more clearly understood from the description and the attached drawing, which shows a practical and non-restrictive example of the invention. In the drawing,

Fig. 1 is a diagram of a device to which the present invention may be applied;
Fig. 2 is a block diagram of the data initialization stage;
Fig. 3 is a graph showing an example of the variation of the estimated temperature;
Fig. 4 is another graph showing an example of the variation of the estimated temperature;
Fig. 5 is a block diagram summarizing the method; and
Figs. 6 and 7 are two detailed block diagrams of two stages of the method summarized in the diagram in Fig. 5.

Detailed description of the preferred embodiment of the invention

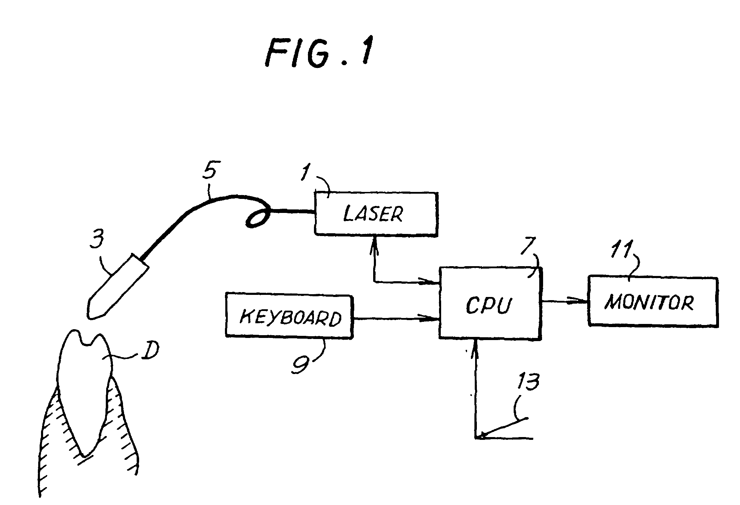

The device to which the invention is applied consists, in outline (see Fig. 1), of a laser source 1 connected to a treatment set 3, for example by means of an optical fiber 5, an optical path with deflecting mirrors and beam guide tubes, or other. The laser source is connected to a programmable central control unit 7, with a keyboard 9 and a monitor 11. The laser source 1 is activated by the operator by means of a suitable control, in this example a control pedal 13. Devices of this type are known and do not require further description.

According to the invention, the central unit 7 is programmable in such a way as to calculate the estimated value of the temperature of the tooth D during the laser treatment, as a function of a set of parameters of the tooth and of the patient undergoing the treatment, and of operating parameters of the device.

The parameters relating to the subject and to the tooth to be treated are: the type of tooth, the size of the tooth and the age of the patient. More particularly, the size of the tooth is defined by a parameter $K_{size}$, which can take the values $K_{size} = 3$, 5 and 7 for small, medium and large teeth respectively, while the type of tooth is defined by a parameter $K_{tooth\_type}$ which takes the following values:

| | |
|---|---|
| incisor: | $K_{tooth\_type} = 92$ |
| canine: | $K_{tooth\_type} = 90$ |
| premolar: | $K_{tooth\_type} = 91$ |
| molar: | $K_{tooth\_type} = 120$ |

Before starting the treatment, in an initialization phase the operator enters the data specified above by means of the keyboard 9, and on the basis of these data the central unit 7 determines a time constant $\tau$ by the following function:

$$\tau = K_{age}\ K_{size}\ K_{tooth\_type} \qquad (1)$$

where $K_{size}$ and $K_{tooth\_type}$ are as defined above and $K_{age}$ is determined as follows:

$$K_{age} = 0.005\ E + 0.80 \qquad (32)$$

where E is the age of the patient, expressed in years.

The time constant $\tau$, expressed in tenths of a second, is used to determine the estimated variation of the temperature inside the tooth when the tooth is struck by the laser beam for the treatment. The equation (1) and the values of the parameters appearing in it, by means of which the time constant $\tau$ is calculated, are determined experimentally. They are therefore only provided by way of example, and may be modified to provide more precise values by a longer experimental phase of data acquisition.

The invention is not, therefore, restricted in any way to these numerical values, which are provided solely by way of example, although they may be considered to have been optimized in the light of present experimental results. It should also be borne in mind that the experimental results on which these values are based were obtained from dead biological material, and were therefore considerably influenced by the quality of the material, particularly its freshness.

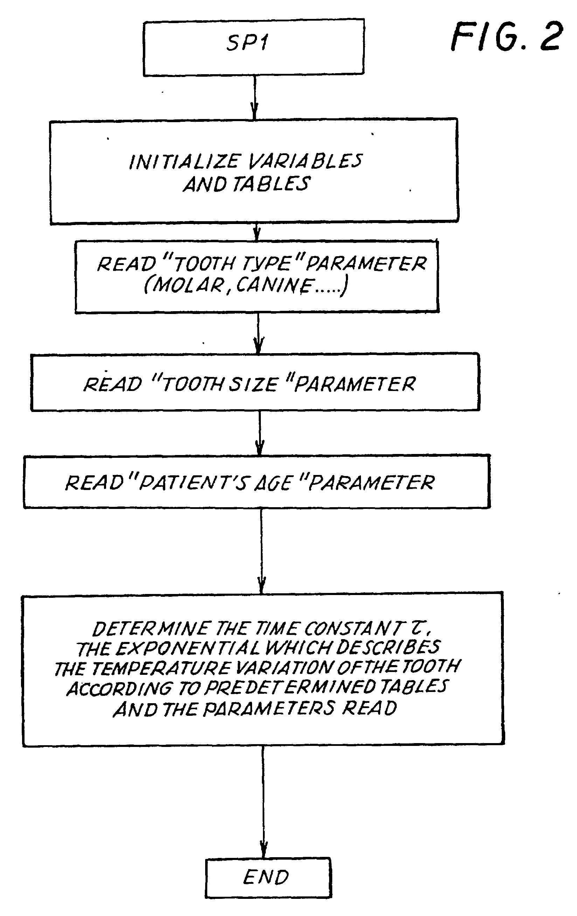

The procedure of initializing the variables described above is summarized schematically in the block diagram in Fig. 2.

The variation of the temperature inside the tooth depends not only on the characteristics of the tooth, according to which the time constant $\tau$ has been determined, but also on the operating conditions, and more particularly on the power of the laser beam, the laser beam emission time, and the initial temperature of the tooth. The variation of the estimated internal temperature of the tooth follows an exponential path which can be approximated with the function:

$$T = T_{max} - (T_{max} - T_{start})\, e^{-t/\tau} \qquad (3)$$

where:

T         is the estimated temperature;

$T_{max}$     is a parameter dependent on the power of the laser beam and possibly also on the characteristics of the tooth, particularly its size. It represents the asymptotic value to which the internal temperature of the tooth tends for a given value of the incident power;

$T_{start}$    is the initial temperature;

t         is the time; and

$\tau$         is the time constant defined above (see Equation 1).

To determine the value of the temperature $T_{max}$, the power W emitted by the laser is initially calculated as the energy E of the laser pulse and the frequency f of repetition of the pulses:

$$W = E\, f \qquad (4)$$

As a first approximation, the temperature $T_{max}$ may be considered to depend only on the power W emitted by the laser, the effect of other parameters, such as the tooth size, being disregarded. It has been found by experimental measurement that the temperature $T_{max}$ can be correlated with the power W of the laser, as shown in Table I below:

Table I

| Power (W) (kW) | $T_{max}$ (°C) |
|---|---|
| 0 | 32 |
| 1 | 40 |
| 2 | 42 |
| 3 | 47 |
| 4 | 53 |
| 5 | 58 |
| 6 | 63 |
| 7 | 68 |
| 8 | 72 |
| 9 | 78 |

In reality, the internal temperature of the tooth is determined by the thermodynamic characteristics of the tooth material, particularly the calorific capacity, and by the balance between the power supplied by the laser and the power dissipated into the environment by the heat dissipation which takes place on the walls of the tooth (in addition to the power removed by the circulation of blood around the tooth and within the tooth, when the tooth is not dead). The dissipation of heat into the environment, in particular, depends on the size of the tooth. This factor may be disregarded as a first approximation, for the reasons which will be made clear subsequently, but it can be taken into consideration by means of an appropriate trial which can be carried out to find the correlation between the temperature of the tooth, the power of the incident laser beam, and the size of the tooth.

Before starting the treatment, the operator enters, through the keyboard 9, the data required by the central unit 7 for the determination of the value of the time constant $\tau$. The device then remains in the waiting state until the treatment starts.

When the laser emission is started, by activation by means of the pedal 13 after the treatment set 3 has been brought to the point where treatment is required, the central unit 7 reads the parameter "f", in other words the emission frequency, in addition to the parameter "E", in other words the energy of the laser pulse, and from these it determines the emission power W (Equation 4). The central unit 7 calculates the value of $T_{max}$ according to Table I, which is made available to the central unit by a suitable storage system, and enables the plotting of a graph on the monitor 11, which represents the variation with time of the function (3), with the time t on the horizontal axis. Before the plotting of the graph is started, the variable $T_{start}$ is given the actual value (for example the base temperature of the tooth, typically assumed to be 32°C), and the emission time t is reset to zero.

The time variable t is then gradually incremented in steps, and at each step the central unit 7 determines the estimated value T of the temperature by the equation (3). In each cycle of the iteration, the estimated temperature T is compared with a threshold alarm value $T_{alarm}$, set, for example, at 40°C. If the value of the estimated temperature T exceeds the value $T_{alarm}$, the central unit emits an alarm signal, for example an acoustic signal, which indicates to the operator the necessity of suspending the treatment to enable the tooth to cool and to prevent the reaching of temperatures which might damage the pulp tissue.

If the estimated value of T at a given step does not exceed the value $T_{alarm}$, the central unit does not emit any alarm. In all cases, it represents the calculated temperature T in graphic form on the monitor 11 and executes the next step of the calculation, after incrementing the time variable (t).

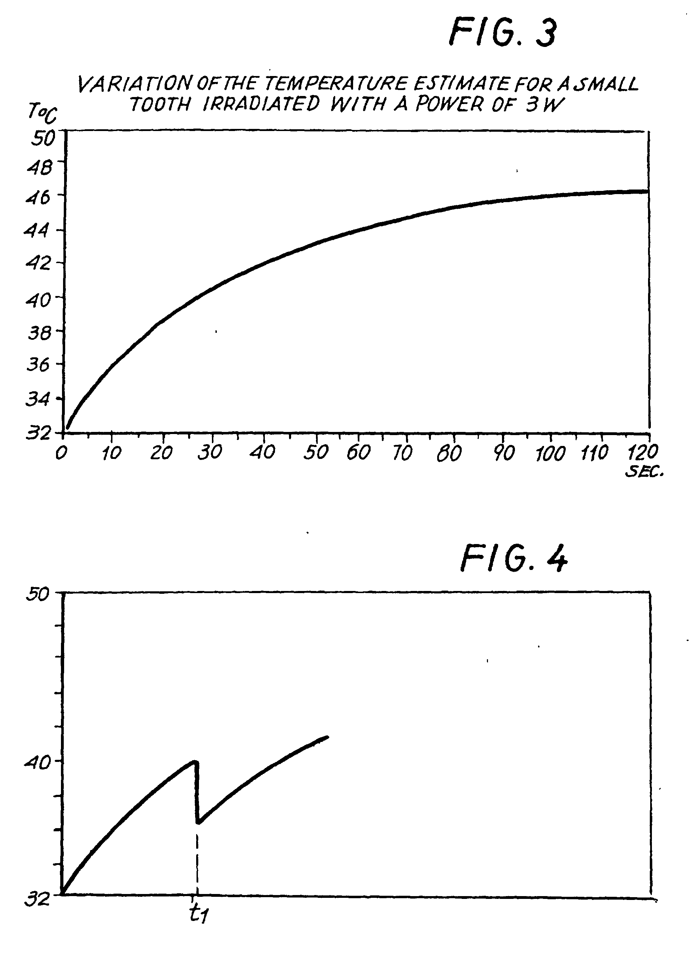

Fig. 3 shows the curve representing the temperature T estimated by this procedure for a small molar (parameters $K_{size}$ = 7; $K_{tooth\_type}$ = 120). The power of the laser is 3 W. The estimated temperature T is shown on the vertical axis and the time t is shown on the horizontal axis.

It is easy to see that the estimated temperature reaches the alarm value at approximately 26 seconds after the start of the emission. If the emission is not interrupted, the (estimated) internal temperature of the tooth increases up to an asymptotic value of approximately 47°C, which is represented by the parameter $T_{max}$. This asymptotic value varies by approximately +/- 20% if the size of the tooth is also taken into consideration in the calculation of the estimated temperature. However, even when this further parameter is taken into consideration, the profile of the lower region of the curve changes to a negligible extent, and therefore the time taken to reach the alarm temperature is substantially independent of the size of the tooth. For this reason, it was specified initially that the temperature $T_{max}$ (which represents the asymptotic value to which the curve of estimated temperature tends) does not have to allow for the size of the tooth.

When, as a result of the emission of an alarm signal, or for any other reason, the operator interrupts the laser emission by releasing the pedal 13, the power W falls to zero and therefore the parameter $T_{max}$ falls to the value of 32°C (base temperature of the tooth). The tooth is at a temperature equal to the last value estimated for the temperature T. This value is then taken by the central unit as the value of $T_{start}$, while the parameter $T_{max}$ takes the value of 32°C (see Table I for W=0).

Given these two values of the parameters $T_{max}$ and $T_{start}$, equation (3) shows that the temperature T has an exponential variation decreasing asymptotically to the value of 32°C. This variation continues until the next start of the laser emission.

The central unit 7 then proceeds (with the new values assigned to the parameters $T_{max}$ and $T_{start}$) to determine the variation of the temperature with time (decreasing) and to represent this variation on the graph on the monitor 11. For reasons of practicality, the movement along the time axis is interrupted for the whole of the time in which the laser emission is equal to zero and the curve representing the estimated temperature T is decreasing. In this way, it is possible to reduce the size of the monitor, or to represent the variation of the temperature T on the monitor for a longer time interval. When the laser is not emitting, the graph on the monitor therefore shows a straight line parallel to the vertical axis, as shown in Fig. 4, where it is assumed that the laser emission is interrupted at the time $t_1$.

When the operator considers that the value of the temperature T has fallen sufficiently below the alarm value $T_{alarm}$, or in any case when the treatment is recommenced, the central unit 7 will again start to detect an exponential increase of the estimated temperature from the value T reached at the instant of starting the second stage of emission. The form of the curve representing T will be rising again, since the parameter $T_{start}$ will take the last estimated value of T at the end of the cooling stage in the absence of emission, and the parameter $T_{max}$ will take the value (Table I) corresponding to the emission power, which will now be different from zero.

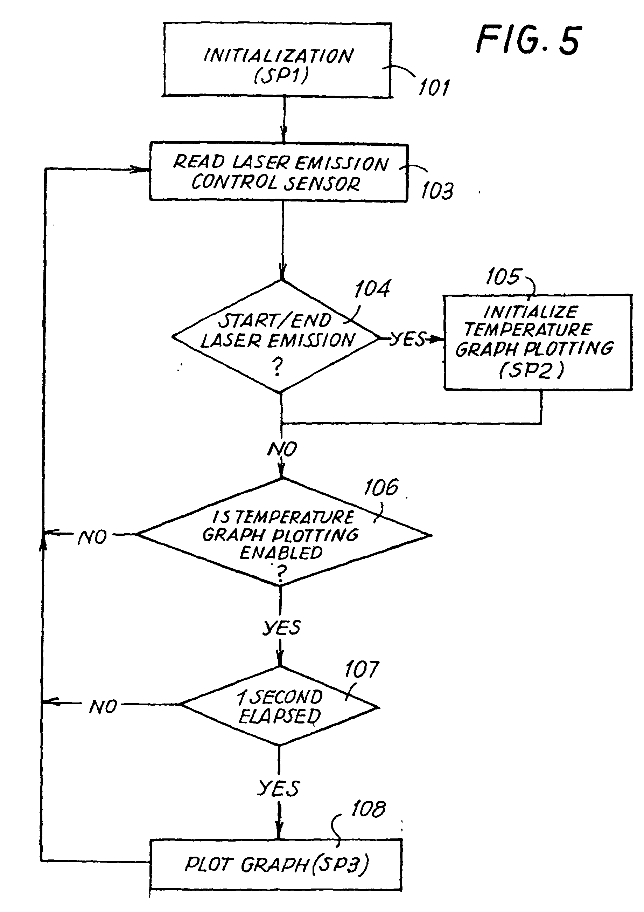

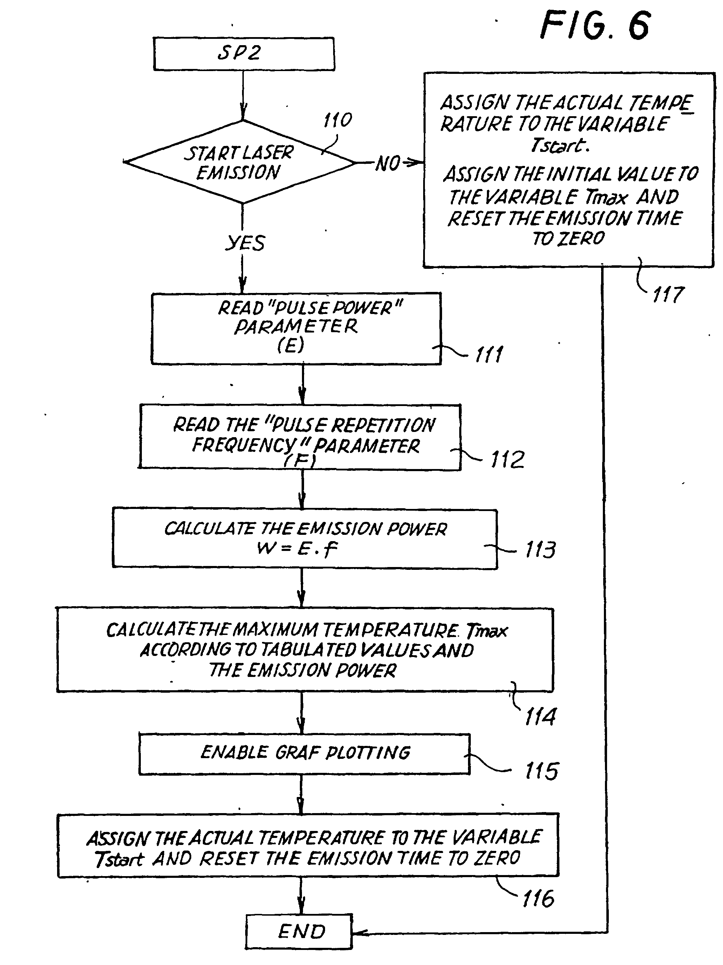

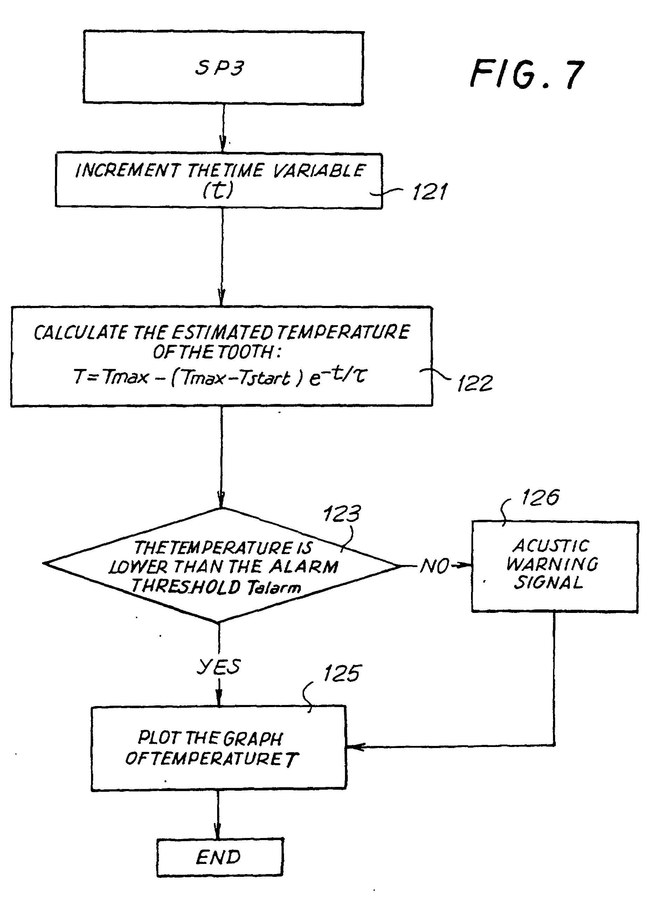

The procedure described above is summarized in the general flow chart in Fig. 5 and in the detailed flow charts in Figs. 6 and 7. In the diagram in Fig. 5, the number 101 indicates the block representing the initialization stage, represented in detail in the diagram in Fig. 2 and described previously. On completion of the initialization stage, the central unit 7 makes cyclic checks to see whether the state of the laser emission controller has been changed, in other words (if the emission has not yet been started) if the laser is still in the non-emitting condition (block 103). If the last monitoring cycle does not find that the laser controller 13 has been activated, the central unit checks whether the plotting of the temperature graph has been enabled (block 106). If the outcome is negative (in other words, if the intervention has not yet been started) the system returns to block 103 and recommences the monitoring cycle.

If the system has already been activated, the checking process moves to block 107 which checks whether more than one second has elapsed since the preceding check. If the outcome is negative, it returns to block 103; if positive, it proceeds to plot the graph (block 108) to show the estimate of the temperature T for the next step, the graph being plotted in steps of 1 second.

If it is found in block 104 that the controller 13 has been activated, this means that the laser has been started or stopped, and that the values of the parameters for the calculation of the estimated temperature T must therefore be changed. If the laser has been started for the first time, the calculation of the function T must be commenced; if the laser has been stopped or restarted after a first stop, the values of the parameters $T_{start}$ and $T_{max}$ must be changed, as described above. For this purpose, the central unit executes block 105, which is shown in detail in the diagram in Fig. 6. An initial check is made to see whether the laser has been activated (start of laser emission = yes) or if it has been stopped (start of laser emission = no). In the first case, the parameter E (energy of the pulse) is read by block 111, and then the frequency parameter "f" is read and the emission power W is calculated (blocks 112, 113) and the value $T_{max}$ is determined from the power W (block 114). The plotting of the graph on the monitor 11 is then enabled (block 115) and the value of the present temperature (for example 32°C, in other words the base temperature of the tooth) is assigned to the variable $T_{start}$, while the time variable t is reset to zero (block 116). The system then returns to the diagram in Fig. 5, at block 106.

If the central unit finds an interruption of the laser emission instead of a start of the laser emission in block 110, it assigns the last calculated value of the temperature T, in other words the actual temperature, to the variable $T_{start}$. The central unit also assigns the initial value, in other words the value corresponding to a zero emission power, to the variable $T_{max}$, and resets the time variable t to zero (block 117). The control process then returns to the diagram in Fig. 5, at block 106.

Fig. 7 shows in detail the procedure for plotting the temperature graph (block 108). The time variable (t) is incremented (block 121). The temperature T is then estimated (block 122) and the estimated value is compared with the value of $T_{alarm}$ (block 123). If the alarm threshold is not exceeded ($T < T_{alarm}$) the central unit shows the newly calculated value of T on the graph displayed on the monitor 11 (block 125) and returns to block 103 (Fig. 5). If the check at block 123 reveals that the estimated temperature T is equal to or greater than the alarm value ($T \geq T_{alarm}$), the system emits an alarm (block 126) and then follows the previously described procedure, passing in all cases to block 125 and from there to block 103 in Fig. 5.

It is to be understood that the drawing shows only an example provided solely as a practical demonstration of the invention, and that this invention may vary in its forms and arrangements without departure from the scope of the guiding concept of the invention. The presence of any reference numbers in the attached claims has the purpose of facilitating the reading of the claims with reference to the description and to the drawing, and does not limit the scope of protection represented by the claims.

**Claims**

1. A method for controlling the internal temperature of a tooth (D) subjected to a treatment by a laser beam during said treatment, wherein an estimated internal temperature (T) of the tooth is determined as a function of, at least, an operating parameter of the laser and the treatment time (t), and wherein the estimated temperature (T) is compared with a threshold temperature ($T_{alarm}$), the exceeding of said threshold temperature generating an alarm condition.

2. The method as claimed in claim 1, wherein said alarm condition comprises the emission of an acoustic signal and/or an optical signal.

3. The method as claimed in claim 1 or 2, wherein said estimated temperature is determined as a function of the emission power (W) of the laser.

4. The method as claimed in claim 1 or 2 or 3, wherein said estimated temperature is determined additionally as a function of the characteristics of the tooth subjected to the laser treatment.

5. The method as claimed in claim 4, wherein said characteristics of the tooth comprise one or more of the following parameters: size, type of tooth, age.

6. The method as claimed in one or more of the preceding claims, wherein said estimated temperature is determined according to the following equation:

$$T = T_{max} - (T_{max} - T_{start})\, e^{-t/\tau} \qquad (3)$$

where:

$t$     is the treatment time;
$T_{start}$     is the initial temperature of the tooth;
$T_{max}$     is a parameter dependent at least on the emission power of the laser;
$\tau$     is a time constant which is a function of the characteristics of the tooth.

7. The method as claimed in claim 6, wherein $T_{max}$ is a parameter dependent on the emission power of the laser and the size of the tooth.

8. The method as claimed in claim 6 or 7, wherein the time constant is determined according to the equation

$$\tau = K_{age}\, K_{size}\, K_{tooth\_type} \qquad (1)$$

where

$K_{age}$     is a constant determined by the age;
$K_{size}$     is a constant determined by the size of the tooth;
$K_{tooth\_type}$     is a constant determined by the type of tooth.

9. The method as claimed in claim 8, wherein $K_{age}$ is defined by

$$K_{age} = 0.005\, E + 0.80 \qquad (2)$$

where E represents the age, expressed in years, of the patient subjected to the treatment.

10. The method as claimed in claim 8 or 9, wherein $K_{size}$ varies from 2 to 10 and preferably from 3 to 7, and increases with the size of the tooth.

11. The method as claimed in claim 8 or 9 or 10, wherein $K_{tooth\_type}$ varies from 70 to 150 and preferably from 90 to 120, as a function of the type of tooth.

12. The method as claimed in claim 11, wherein $K_{tooth\_type}$ takes the following values as a function of the type of tooth:

incisor:     $K_{tooth\_type} = 92$
canine:     $K_{tooth\_type} = 90$
premolar:     $K_{tooth\_type} = 91$
molar:     $K_{tooth\_type} = 120$

13. The method as claimed in one or more of the preceding claims, wherein the estimated temperature is shown on a graph as a function of the treatment time.

14. The method as claimed in claim 13, wherein during the stages of interruption of the laser emission, in which the estimated temperature decreases, the variation of the estimated temperature is shown on the graph as moving along the vertical axis but without movement along the horizontal axis.

15. A device for dental treatment by means of a laser beam, including a laser source (1), a treatment set (3) for treating a tooth (D), a central control unit (7) connected to said laser source and to means (13) of turning the laser beam on and off, **characterized in that** said central unit is programmed to apply a method as claimed in one or more of claims 1 to 14.

16. A device for dental treatment by means of a laser beam, including a laser source (1), a treatment set (3) for treating a tooth (D), a central control unit (7) connected to said laser source and to means (13) of turning the laser beam on and off, **characterized in that** said central unit comprises means for determining an estimated internal temperature (T) of the tooth subjected to the treatment by means of a beam emitted by said laser source as a function of, at least, an operating parameter of the laser and the treatment time (t), said central unit comparing said estimated temperature (T) with a threshold temperature ($T_{alarm}$), the exceeding of said threshold temperature causing the generation of an alarm signal.

17. The device as claimed in claim 16, wherein said central unit determines the estimated temperature as a function of the emission power (W) of said laser source (1).

18. The device as claimed in claim 16 or 17, **characterized in that** said central unit (7) is programmed to determine said estimated temperature (T) additionally as a function of the characteristics of the tooth subjected to the laser treatment.

19. The device as claimed in one or more of claims 16 to 18, **characterized in that** said central unit is programmed to determine said estimated temperature (T) as a function of one or more of the following parameters: size of the tooth, type of tooth, age.

20. The device as claimed in one or more of claims 16 to 19, **characterized in that** said central unit is programmed to determine said estimated temperature according to the following equation:

$$T = T_{max} - (T_{max} - T_{start})\, e^{-t/\tau} \tag{3}$$

where:

t         is the treatment time;
$T_{start}$    is the initial temperature of the tooth;
$T_{max}$    is a parameter dependent at least on the emission power of the laser;
$\tau$         is a time constant which is a function of the characteristics of the tooth.

21. The device as claimed in claim 20, **characterized in that** $T_{max}$ is a parameter dependent on the emission power of the laser source and on the size of the tooth.

22. The device as claimed in claim 20 or 21, **characterized in that** the central unit determines the time constant $\tau$ according to the equation

$$\tau = K_{age}\, K_{size}\, K_{tooth\_type} \tag{1}$$

where

$K_{age}$       is a constant determined by the age;
$K_{size}$       is a constant determined by the size of the tooth;
$K_{tooth\_type}$   is a constant determined by the type of tooth.

23. The device as claimed in claim 22, **characterized in that** $K_{age}$ is defined by

$$K_{age} = 0.005\, E + 0.80 \tag{2}$$

where E represents the age, expressed in years, of the patient subjected to the treatment.

24. The device as claimed in claim 22 or 23, in which $K_{size}$ varies from 2 to 10 and preferably from 3 to 7, and increases with the size of the tooth.

25. The device as claimed in claim 22 or 23 or 24, **characterized in that** $K_{tooth\_type}$ varies from 70 to 150 and preferably from 90 to 120, as a function of the type of tooth.

26. The device as claimed in claim 25, in which $K_{tooth\_type}$ takes the following values as a function of the type of tooth:

incisor: $K_{tooth\_type} = 92$
canine: $K_{tooth\_type} = 90$
premolar: $K_{tooth\_type} = 91$
molar: $K_{tooth\_type} = 120$

27. The device as claimed in one or more of claims 16 to 26, **characterized in that** it comprises a display means (11) on which is displayed a graph showing the variation of the estimated temperature as a function of the treatment time.

28. The device as claimed in claim 27, **characterized in that** said central unit (7) is programmed in such a way that during the stages of interruption of the laser emission, in which the estimated temperature decreases, the variation of the estimated temperature is shown on the graph as moving along the vertical axis but without movement along the horizontal axis.

## Patentansprüche

1. Verfahren zur Kontrolle der inneren Temperatur eines einer Behandlung durch einen Laserstrahl unterzogenen Zahnes (D) während der genannten Behandlung, wobei eine geschätzte innere Temperatur (T) des Zahnes als Funktion von mindestens einem Betriebsparameter des Lasers und der Behandlungszeit (t) bestimmt wird und wobei die geschätzte Temperatur (T) mit einer Schwellwerttemperatur ($T_{Alarm}$) verglichen wird, wobei das Überschreiten der genannten Schwellwerttemperatur eine Alarmbedingung erzeugt.

2. Verfahren gemäß Anspruch 1, wobei die genannte Alarmbedingung die Abgabe eines akustischen und/oder optischen Signals umfasst.

3. Verfahren gemäß Anspruch 1 oder 2, wobei die genannte geschätzte Temperatur als Funktion der Emissionsleistung (W) des Lasers bestimmt wird.

4. Verfahren gemäß Anspruch 1 oder 2 oder 3, wobei die genannte geschätzte Temperatur zusätzlich als Funktion der charakteristischen Merkmale des Zahnes, welcher der Laserbehandlung unterzogen wird, bestimmt wird.

5. Verfahren gemäß Anspruch 4, wobei die genannten charakteristischen Merkmale des Zahnes einen oder mehrere der folgenden Parameter umfasst: Größe, Art des Zahnes, Alter.

6. Verfahren gemäß einem oder mehreren der vorangegangenen Ansprüche, wobei die genannte geschätzte Temperatur gemäß der folgenden Gleichung bestimmt wird:

$$T = T_{max} - (T_{max} - T_{Start})\, e^{-t/\tau} \tag{3}$$

worin:

t  die Behandlungszeit ist;
$T_{Start}$  die Anfangstemperatur des Zahnes ist;
$T_{max}$  ein Parameter ist, welcher zumindest von der Emissionsleistung des Lasers abhängt;
$\tau$  eine Zeitkonstante ist, welche eine Funktion der charakteristischen Merkmale des Zahnes darstellt.

7. Verfahren gemäß Anspruch 6, wobei $T_{max}$ einen Parameter darstellt, welcher von der Emissionsleistung des Lasers und der Größe des Zahnes abhängt.

8. Verfahren gemäß Anspruch 6 oder 7, wobei die Zeit konstante gemäß der Gleichung

$$\tau = K_{Alter} * K_{Größe} * K_{Zahnart} \tag{1}$$

bestimmt wird, worin

$K_{Alter}$ eine durch das Alter bestimmte Konstante ist;
$K_{Größe}$ eine durch die Größe des Zahnes bestimmte Konstante ist;
$K_{Zahnart}$ eine durch die Art des Zahnes bestimmte Konstante ist.

9. Verfahren gemäß Anspruch 8, wobei $K_{Alter}$ durch

$$K_{Alter} = 0,005\, E + 0,80 \tag{2}$$

definiert wird, worin E das in Jahren ausgedrückte Alter des Patienten darstellt; welcher der Behandlung unterzogen wird.

10. Verfahren gemäß Anspruch 8 oder 9, wobei $K_{Größe}$ von 2 bis 10, vorzugsweise von 3 bis 7, variiert und mit der Größe des Zahnes ansteigt.

11. Verfahren gemäß Anspruch 8 oder 9 oder 10, wobei $K_{Zahnart}$ als eine Funktion der Art des Zahnes von 70 bis 150, vorzugsweise von 90 bis 120, variiert.

12. Verfahren gemäß Anspruch 11, wobei $K_{Zahnart}$ die folgenden Werte als eine Funktion der Art des Zahnes annimmt:

| | |
|---|---|
| Schneidezahn: | $K_{Zahnart} = 92$ |
| Eckzahn: | $K_{Zahnart} = 90$ |
| vorderer Backenzahn: | $K_{Zahnart} = 91$ |
| Backenzahn: | $K_{Zahnart} = 120$ |

13. Verfahren gemäß einem oder mehreren der vorangegangenen Ansprüche, wobei die geschätzte Temperatur auf einem Diagramm als Funktion der Behandlungszeit dargestellt wird.

14. Verfahren gemäß Anspruch 13, wobei während der Stufen der Unterbrechung der Laseremission, in welchen die geschätzte Temperatur absinkt, die Änderung der geschätzten Temperatur auf dem Diagramm als Bewegung längs der vertikalen Achse, aber ohne Bewegung längs der horizontalen Achse dargestellt wird.

15. Vorrichtung zur zahnärztlichen Behandlung mittels eines Laserstrahles, welche enthält: eine Laserquelle (1), eine Behandlungsgarnitur (3) für die Behandlung des Zahnes (D) und eine zentrale Steuereinheit (7), welche an die genannte Laserquelle und an Mittel (13) für das Ein- und Ausschalten des Lasers angeschlossen ist, **dadurch gekennzeichnet, dass** die genannte zentrale Einheit programmiert ist, um ein Verfahren anzuwenden, welches in einem oder mehreren der Ansprüche 1 bis 14 beansprucht wird.

16. Vorrichtung zur zahnärztlichen Behandlung mittels eines Laserstrahles, welche enthält: eine Laserquelle (1), eine Behandlungsgarnitur (3) für die Behandlung des Zahnes (D) und eine zentrale Steuereinheit (7), welche an die genannte Laserquelle und an Mittel (13) für das Ein- und Ausschalten des Lasers angeschlossen ist, **dadurch gekennzeichnet, dass** die genannte zentrale Einheit Mittel umfasst zur Bestimmung einer geschätzten inneren Temperatur (T) des Zahnes, welcher der Behandlung mit einem von der genannten Laserquelle ausgesandten Strahl unterzogen wird, als Funktion von mindestens einem Betriebsparameters des Lasers und der Behandlungszeit (t), wobei die genannte zentrale Einheit die genannte geschätzte Temperatur (T) mit einer Schwellwerttemperatur ($T_{Alarm}$) vergleicht, wobei das Überschreiten der genannten Schwellwerttemperatur die Erzeugung eines Alarmsignals bewirkt.

17. Vorrichtung gemäß Anspruch 16, bei welcher die genannte zentrale Einheit die geschätzte Temperatur als Funktion der Emissionsleistung (W) der genannten Laserquelle (1) bestimmt.

**18.** Vorrichtung gemäß Anspruch 16 oder 17, **dadurch gekennzeichnet, dass** die zentrale Einheit (7) programmiert wird, um die genannte geschätzte Temperatur (T) zusätzlich als Funktion der charakteristischen Merkmale des der Laserbehandlung unterzogenen Zahnes zu bestimmen.

**19.** Vorrichtung gemäß einem oder mehreren der Ansprüche 16 bis 18, **dadurch gekennzeichnet, dass** die genannte zentrale Einheit programmiert wird, um die genannte geschätzte Temperatur (T) als Funktion von einem oder mehreren der folgenden Parameter zu bestimmen: Größe des Zahnes, Art des Zahnes, Alter.

**20.** Vorrichtung gemäß einem oder mehreren der Ansprüche 16 bis 19, **dadurch gekennzeichnet, dass** die genannte zentrale Einheit programmiert wird, um die genannte geschätzte Temperatur gemäß der folgenden Gleichung zu bestimmen:

$$T = T_{max} - (T_{max} - T_{Start})\, e^{-t/\tau} \tag{3}$$

worin:

$t$     die Behandlungszeit ist;
$T_{Start}$     die Anfangstemperatur des Zahnes ist;
$T_{max}$     ein Parameter ist, welcher zumindest von der Emissionsleistung des Lasers abhängt;
$\tau$     eine Zeitkonstante ist, welche eine Funktion der charakteristischen Merkmale des Zahnes darstellt.

**21.** Vorrichtung gemäß Anspruch 20, **dadurch gekennzeichnet, dass** $T_{max}$ einen Parameter darstellt, welcher von der Emissionsleistung des Lasers und von der Größe des Zahnes abhängt.

**22.** Vorrichtung gemäß Anspruch 20 oder 21, **dadurch gekennzeichnet, dass** die zentrale Einheit die Zeitkonstante gemäß der Gleichung

$$\tau = K_{Alter} * K_{Größe} * K_{Zahnart} \tag{1}$$

bestimmt, worin

$K_{Alter}$ eine durch das Alter bestimmte. Konstante ist;
$K_{Größe}$ eine durch die Größe des Zahnes bestimmte Konstante ist;
$K_{Zahnart}$ eine durch die Art des Zahnes bestimmte Konstante ist.

**23.** Vorrichtung gemäß Anspruch 22, **dadurch gekennzeichnet, dass** $K_{Alter}$ durch

$$K_{Alter} = 0{,}005\, E + 0{,}80 \tag{2}$$

definiert ist, worin E das in Jahren ausgedrückte Alter des Patienten darstellt, welcher der Behandlung unterzogen wird.

**24.** Vorrichtung gemäß Anspruch 22 oder 23, in welcher $K_{Größe}$ von 2 bis 10, vorzugsweise von 3 bis 7, variiert und mit der Größe des Zahnes ansteigt.

**25.** Vorrichtung gemäß Anspruch 22 oder 23 oder 24, **dadurch gekennzeichnet, dass** $K_{Zahnart}$ als Funktion der Art des Zahnes von 70 bis 150, vorzugsweise von 90 bis 120, variiert.

**26.** Vorrichtung gemäß Anspruch 25, in welcher $K_{Zahnart}$ die folgenden Werte als Funktion der Art des Zahnes annimmt:

Schneidezahn:     $K_{Zahnart} = 92$
Eckzahn:     $K_{Zahnart} = 90$
vorderer Backenzahn:     $K_{Zahnart} = 91$
Backenzahn:     $K_{Zahnart} = 120$

**27.** Vorrichtung gemäß einem oder mehreren der Ansprüche 16 bis 26, **dadurch gekennzeichnet, dass** sie ein Displaymittel (11) umfasst, auf welchem ein Diagramm dargestellt wird, welches die Änderung der geschätzten Temperatur als Funktion der Behandlungszeit darstellt.

**28.** Vorrichtung gemäß Anspruch 27, **dadurch gekennzeichnet, dass** die genannte zentrale Einheit (7) dergestalt programmiert ist, dass während der Stufen der Unterbrechung der Laseremission, in welchen die geschätzte Temperatur absinkt, die Änderung der geschätzten Temperatur auf dem Diagramm als Bewegung längs der vertikalen Achse, aber ohne Bewegung längs der horizontalen Achse dargestellt wird.

**Revendications**

**1.** Procédé de contrôle de la température interne d'une dent (D) soumise à un traitement par un faisceau laser pendant ledit traitement, dans lequel une température interne estimée (T) de la dent est déterminée en fonction d'au moins un paramètre de service du laser et du temps de traitement (t), et dans lequel la température estimée (T) est comparée à une température de seuil (Talarme), le dépassement de la dite température de seuil générant une condition d'alarme.

**2.** Procédé selon la revendication 1, dans lequel ladite condition d'alarme comprend l'émission d'un signal acoustique et/ou d'un signal optique.

**3.** Procédé selon la revendication 1 ou 2, dans lequel ladite température estimée est déterminée en fonction de la puissance d'émission (W) du laser.

**4.** Procédé selon la revendication 1 ou 2 ou 3, dans lequel ladite température estimée est déterminée de façon supplémentaire en fonction des caractéristiques de la dent soumise au traitement au laser.

**5.** Procédé selon la revendication 4, dans lequel lesdites caractéristiques de la dent comprennent un ou plusieurs des paramètres suivants : taille, type de dent, âge.

**6.** Procédé selon l'une ou plusieurs des revendications précédentes, dans lequel ladite température estimée est déterminée selon l'équation suivante :

$$T = T_{max} - (T_{max} - T_{début})\, e^{-t/\tau} \qquad (3)$$

dans laquelle :

t est le temps de traitement ;
$T_{début}$ est la température initiale de la dent ;
$T_{max}$ est un paramètre dépendant au moins de la puissance d'émission du laser ;
$\tau$ est une constante de temps qui dépend des caractéristiques de la dent.

**7.** Procédé selon la revendication 6, dans lequel $T_{max}$ est un paramètre dépendant de la puissance d'émission du laser et de la taille de la dent.

**8.** Procédé selon la revendication 6 ou 7, dans lequel la constante de temps est déterminée selon l'équation suivante

$$\tau = K_{âge}\, K_{taille}\, K_{type\_dent} \qquad (1)$$

dans laquelle :

$K_{âge}$ est une constante déterminée par l'âge ;
$K_{taille}$ est une constante déterminée par la taille de la dent ;
$K_{type\_dent}$ est une constante déterminée par le type de dent.

**9.** Procédé selon la revendication 8, dans lequel $K_{\hat{a}ge}$ est défini par

$$K_{\hat{a}ge} = 0,005 \, E + 0,80 \qquad\qquad (2)$$

où E représente l'âge, exprimé en années, du patient soumis au traitement.

**10.** Procédé selon la revendication 8 ou 9, dans lequel $K_{taille}$ varie de 2 à 10 et de préférence de 3 à 7, et augmente avec la taille de la dent.

**11.** Procédé selon la revendication 8 ou 9 ou 10, dans lequel $K_{type\_dent}$ varie de 70 à 150 et de préférence de 90 à 120, en fonction du type de dent.

**12.** Procédé selon la revendication 11, dans lequel $K_{type\_dent}$ prend les valeurs suivantes en fonction du type de dent :

incisive : $K_{type\_dent} = 92$
canine : $K_{type\_dent} = 90$
prémolaire : $K_{type\_dent} = 91$
molaire : $K_{type\_dent} = 120$

**13.** Procédé selon l'une ou plusieurs des revendications précédentes, dans lequel la température estimée est illustrée sur un graphique en fonction du temps de traitement.

**14.** Procédé selon la revendication 13, dans lequel pendant les étapes d'interruption de l'émission du laser, pendant lesquelles la température estimée diminue, la variation de la température estimée est illustrée sur le graphique comme se déplaçant le long de l'axe vertical mais sans déplacement le long de l'axe horizontal.

**15.** Dispositif pour le traitement dentaire au moyen d'un faisceau laser, comprenant une source de laser (1), un instrument de traitement (3) pour traiter une dent (D), une unité de commande centrale (7) connectée à ladite source de laser et à des moyens (13) de mise en service et hors service du faisceau laser, **caractérisé en ce que** ladite unité centrale est programmée pour appliquer un procédé selon l'une ou plusieurs des revendications 1 à 14.

**16.** Dispositif pour le traitement dentaire au moyen d'un faisceau laser, comprenant une source de laser (1), un instrument de traitement (3) pour traiter une dent (D), une unité de commande centrale (7) connectée à ladite source de laser et à des moyens (13) de mise en service et hors service du faisceau laser, **caractérisé en ce que** ladite unité centrale comprend des moyens pour déterminer une température interne estimée (T) de la dent soumise au traitement au moyen d'un faisceau émis par ladite source de laser en fonction, au moins, d'un paramètre de service du laser et du temps de traitement (t), ladite unité centrale comparant ladite température estimée (T) avec une température de seuil ($T_{alarme}$), le dépassement de ladite température de seuil entraînant la génération d'un signal d'alarme.

**17.** Dispositif selon la revendication 16, dans lequel ladite unité centrale détermine la température estimée en fonction de la puissance d'émission (W) de ladite source de laser (1).

**18.** Dispositif selon la revendication 16 ou 17, **caractérisé en ce que** ladite unité centrale (7) est programmée pour déterminer ladite température estimée (T) de façon supplémentaire en fonction des caractéristiques de la dent soumise au traitement au laser.

**19.** Dispositif selon l'une ou plusieurs des revendications 16 à 18, **caractérisé en ce que** ladite unité centrale est programmée pour déterminer ladite température estimée (T) en fonction d'un ou de plusieurs des paramètres suivants : taille de la dent, type de dent, âge.

**20.** Dispositif selon l'une ou plusieurs des revendications 16 à 19, **caractérisé en ce que** ladite unité centrale est programmée pour déterminer ladite température estimée selon l'équation suivante :

$$T = T_{max} - (T_{max} - T_{début}) \, e^{-t/\tau} \qquad\qquad (3)$$

dans laquelle :

| | |
|---|---|
| t | est le temps de traitement ; |
| $T_{début}$ | est la température initiale de la dent ; |
| $T_{max}$ | est un paramètre dépendant au moins de la puissance d'émission du laser ; |
| $\tau$ | est une constante de temps qui dépend des caractéristiques de la dent. |

**21.** Dispositif selon la revendication 20, **caractérisé en ce que** $T_{max}$ est un paramètre dépendant de la puissance d'émission de la source de laser et de la taille de la dent.

**22.** Dispositif selon la revendication 20 ou 21, **caractérisé en ce que** l'unité centrale détermine la constante de temps $\tau$ selon l'équation

$$\tau = K_{âge} \, K_{taille} \, K_{type\_dent} \qquad (1)$$

dans laquelle :

| | |
|---|---|
| $K_{âge}$ | est une constante déterminée par l'âge ; |
| $K_{taille}$ | est une constante déterminée par la taille de la dent ; |
| $K_{type\_dent}$ | est une constante déterminée par le type de dent. |

**23.** Dispositif selon la revendication 22, **caractérisé en ce que** $K_{âge}$ est défini par

$$K_{âge} = 0{,}0005 \, E + 0{,}80 \qquad (2)$$

où E représente l'âge, exprimé en années, du patient soumis au traitement.

**24.** Dispositif selon la revendication 22 ou 23, dans lequel $K_{taille}$ varie de 2 à 10 et de préférence de 3 à 7, et augmente avec la taille de la dent.

**25.** Dispositif selon la revendication 22 ou 23 ou 24, **caractérisé en ce que** $K_{type\_dent}$ varie de 70 à 150 et de préférence de 90 à 120, en fonction du type de dent.

**26.** Dispositif selon la revendication 25, dans lequel $K_{type\_dent}$ prend les valeurs suivantes en fonction du type de dent :

| | |
|---|---|
| incisive : | $K_{type\_dent} = 92$ |
| canine : | $K_{type\_dent} = 90$ |
| prémolaire : | $K_{type\_dent} = 91$ |
| molaire : | $K_{type\_dent} = 120$ |

**27.** Dispositif selon l'une ou plusieurs des revendications 16 à 26, **caractérisé en ce qu'**il comprend un moyen d'affichage (11) sur lequel s'affiche un graphique représentant la variation de la température estimée en fonction du temps de traitement.

**28.** Dispositif selon la revendication 27, **caractérisé en ce que** ladite unité centrale (7) est programmée d'une telle manière que pendant les étapes d'interruption de l'émission du laser, pendant lesquelles la température estimée diminue, la variation de la température estimée est illustrée sur le graphique comme se déplaçant le long de l'axe vertical mais sans déplacement le long de l'axe horizontal.

# FIG.1

FIG. 2

SP1

INITIALIZE VARIABLES
AND TABLES

READ "TOOTH TYPE" PARAMETER
(MOLAR, CANINE.....)

READ "TOOTH SIZE" PARAMETER

READ "PATIENT'S AGE" PARAMETER

DETERMINE THE TIME CONSTANT $\tau$,
THE EXPONENTIAL WHICH DESCRIBES
THE TEMPERATURE VARIATION OF THE TOOTH
ACCORDING TO PREDETERMINED TABLES
AND THE PARAMETERS READ

END

## FIG. 3

VARIATION OF THE TEMPERATURE ESTIMATE FOR A SMALL
TOOTH IRRADIATED WITH A POWER OF 3 W

## FIG. 4

FIG. 5

INITIALIZATION (SP1) — 101

READ LASER EMISSION CONTROL SENSOR — 103

START/END LASER EMISSION? — 104 → YES → INITIALIZE TEMPERATURE GRAPH PLOTTING (SP2) — 105

NO

IS TEMPERATURE GRAPH PLOTTING ENABLED? — 106 → NO

YES

1 SECOND ELAPSED — 107 → NO

YES

PLOT GRAPH (SP3) — 108

## FIG. 6

```
SP2
```

↓

START LASER EMISSION  *110*  ——NO—→  ASSIGN THE ACTUAL TEMPE‐ RATURE TO THE VARIABLE *Tstart*.
ASSIGN THE INITIAL VALUE TO THE VARIABLE *Tmax* AND RESET THE EMISSION TIME TO ZERO  *117*

↓ YES

READ "PULSE POWER" PARAMETER (E)  *111*

↓

READ THE "PULSE REPETITION FREQUENCY" PARAMETER (F)  *112*

↓

CALCULATE THE EMISSION POWER $W = E \cdot f$  *113*

↓

CALCULATE THE MAXIMUM TEMPERATURE. *Tmax* ACCORDING TO TABULATED VALUES AND THE EMISSION POWER  *114*

↓

ENABLE GRAF PLOTTING  *115*

↓

ASSIGN THE ACTUAL TEMPERATURE TO THE VARIABLE *Tstart* AND RESET THE EMISSION TIME TO ZERO  *116*

↓

```
END
```

FIG. 7

```
┌─────────────────────────┐
│          S P 3          │
└─────────────────────────┘
             │
             ▼
┌─────────────────────────┐
│ INCREMENT THE TIME      │ ╮ 121
│ VARIABLE (t)            │ ╯
└─────────────────────────┘
             │
             ▼
┌───────────────────────────────────────┐
│ CALCULATE THE ESTIMATED TEMPERATURE    │
│ OF THE TOOTH:                          │ ╮ 122
│ T = Tmax − (Tmax − Tstart) e^{−t/τ}    │ ╯
└───────────────────────────────────────┘
             │
             ▼
          123 ╮
        ◇ THE TEMPERATURE IS ◇──── NO ───▶ ┌──────────────┐  ╮ 126
        ◇ LOWER THAN THE ALARM ◇           │   ACUSTIC    │
        ◇ THRESHOLD Talarm ◇               │   WARNING    │
             │                             │   SIGNAL     │
            YES                            └──────────────┘
             │                                    │
             ▼                                    │
┌─────────────────────────┐ ╮ 125               │
│ PLOT THE GRAPH          │ ◀──────────────────┘
│ OF TEMPERATURE T        │
└─────────────────────────┘
             │
             ▼
      ┌───────────┐
      │    END    │
      └───────────┘
```

$$T = Tmax - (Tmax - Tstart)\, e^{-t/\tau}$$